# EUROPEAN PATENT APPLICATION

(11) **EP 4 299 753 A1**
(43) Date of publication of application: **03.01.2024**
(21) Application number: 22181773.7
(22) Date of filing: 29.06.2022
(51) Int. Cl.: C12P 19/34, C12N 1/20

(54) **METHOD FOR PRODUCING EXTRACHROMOSOMAL NUCLEIC ACIDS**

(71) Applicant: enGenes Biotech GmbH, 1190 Wien (AT)
(72) Inventor: MAIRHOFER, Jürgen, 1170 Wien (AT); STROBL, Florian, 2124 Niederkreuzstetten (AT); WEIß, Florian, 3702 Oberrußbach (AT); ZANGHELLINI, Jürgen, 3340 Waidhofen/Ybbs (AT); GOTSMY, Mathias, 1170 Wien (AT)
(74) Representative: Schiweck Weinzierl Koch Patentanwälte Partnerschaft mbB

(57) **Abstract**

The present invention relates to a method for increasing the production of a nucleic acid comprising the step of limiting the total sulfur amount in the culture medium. The present invention further relates to the use of a culture medium comprising a limited sulfur amount for increasing the production of a nucleic acid.

## Description

The present invention is in the field of recombinant biotechnology, in particular in the field of nucleic acid production. The present invention relates to a method for increasing the production of a nucleic acid comprising the step of limiting the total sulfur amount in the culture medium. The present invention further relates to the use of a culture medium comprising a limited sulfur amount for increasing the production of a nucleic acid.

Production of proteins of interest (POI) or nucleic acid molecules of interest has been accomplished with many prokaryotic hosts. The most prominent examples are bacteria like *Escherichia coli, Bacillus subtilis, Pseudomonas fluorescens, Streptomyces griseus,* or *Corynebacterium glutamicum.* A great number of biological pharmaceuticals (e.g. antibodies or functional fragments thereof, or nucleic acid molecules) have been produced in the last years and an increasing number is nearing approval for use in humans but their efficient production remains a challenging task. Therapeutically active doses for an increasing number of people in need thereof require substantial amounts of such protein or nucleic acid molecules. Also, as nucleic acid molecules are needed to produce, e.g. proteins in production cells, plasmids for vaccination, rAAV vectors, etc. a substantial amount of such nucleic acid molecules is needed. Thus, considerable amounts of molecules are needed as active ingredients or as templates for protein production, making an efficient and cost-effective production worthwhile.

Bacterial cell expression systems have long been, and still are, one of the major tools for production of these types of molecules. The key objective of process optimization is to achieve a high yield of product having the required quality at the lowest possible cost, which is often determined by the properties of a specific expression construct or system. Very often, a batch process is used for, e.g. nucleic acid production or to produce plasmids for vaccination, such as AAV vectors, etc. A batch process however is labor-intensive because the bioreactors require constant surveillance and must be cleaned thoroughly after each production. While the bioreactors are cleaned, production cannot take place making batch processes inefficient.

By applying a continuous expression system instead of a batch process, these disadvantages associated with a batch process can be overcome. However, also in a continuous process, high-level recombinant nucleic acid molecule synthesis may overwhelm the metabolic capacity of a host cell and consequently leads to plasmid loss, reduced oxygen transfer, generation of toxic by-products, formation of inclusion bodies, and/or triggering of a stress response, which often impairs efficient nucleic acid molecule synthesis.

To this end, different approaches have been taken by scientists to deal with these problems when using a batch process or a continuous process. Nevertheless, there is still a need for an effective production of proteins or nucleic acid molecules of interest in microbial host cells.

The described and further disadvantages need to be overcome. The present invention therefore addresses these needs and technical objectives and provides a solution as described herein and as defined in the claims.

The present invention relates to a method for increasing the production of a nucleic acid (preferably extrachromosomal nucleic acid) comprised by a host cell in cell culture, comprising the step of limiting over the course of cell culturing the total sulfur amount in the culture medium of said cell culture such that the total sulfur amount is per each gram predetermined dry cell weight of said host cell, which is desired to be obtained in said cell culture, about equal to or less than the amount of sulfur contained per each gram dry weight of a reference host cell, preferably when grown without limiting the total sulfur amount, thereby increasing the production of said nucleic acid.

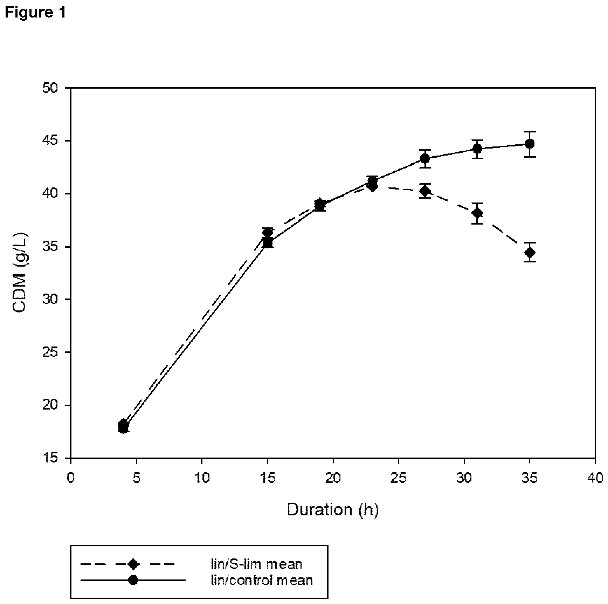

As is demonstrated in the Examples and shown in the Figures, but without being limited thereto, the methods of the present invention provide for decoupling metabolism of a host cell from the production of a desired nucleic acid, e.g. a plasmid. As soon as sulfur becomes limiting, host cells, preferably bacterial cells, will no longer be able to synthesize biomass, but produce a desired nucleic acid, e.g. a plasmid; see also Figure 1.

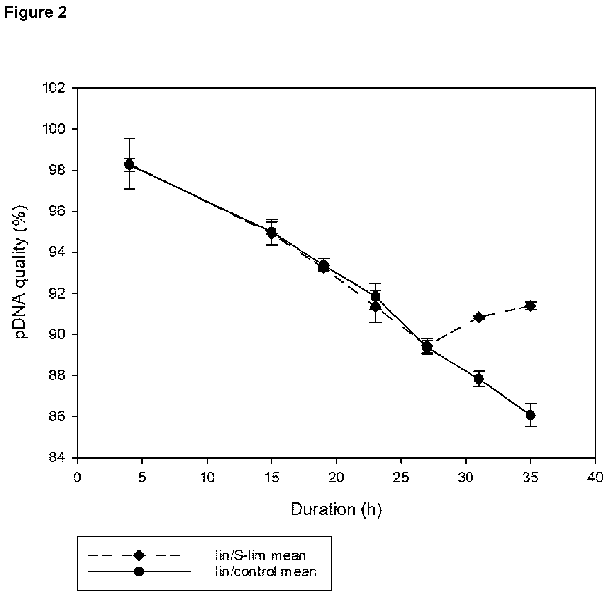

Surprisingly, when performing the methods of the present invention, it was observed that the quality of the desired nucleic acid, preferably pDNA, produced by a culture under S-limitation increases when sulfur limitation occurs; see Figure 2.

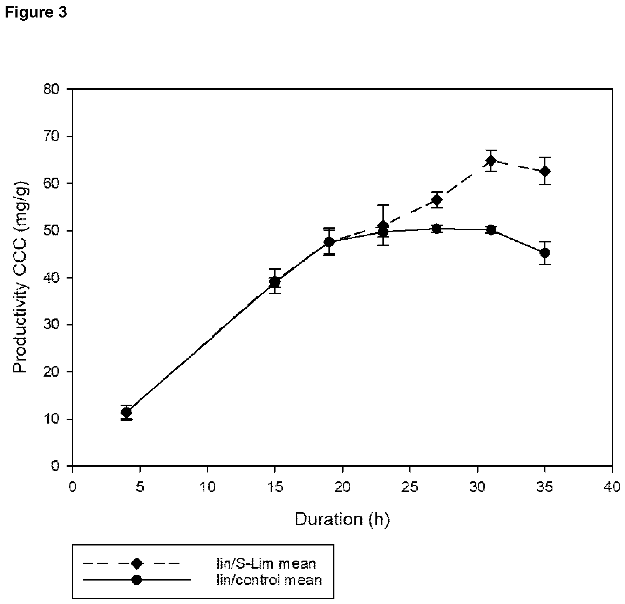

Similarly, when performing the methods of the present invention, it was observed that the production of the desired nucleic acid, preferably ccc (covalently, closed, circular) DNA productivity, by a culture under S-limitation increases when sulfur limitation occurs; see Figure 3.

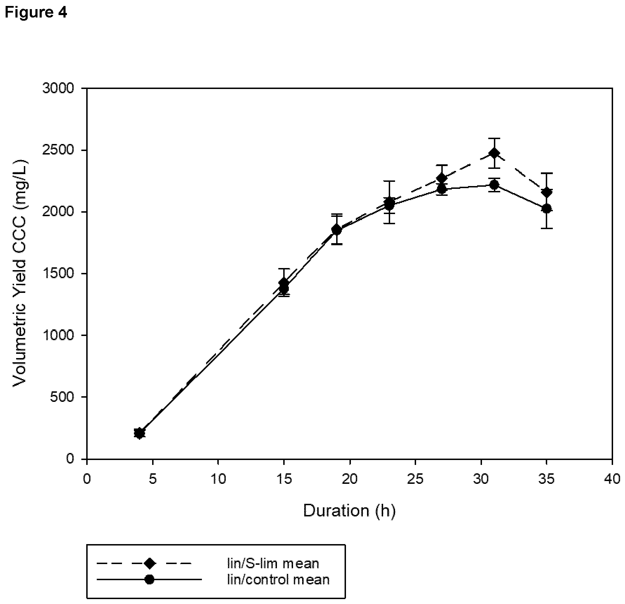

Also, when performing the methods of the present invention, it was observed that the yield of the desired nucleic acid, preferably ccc DNA yield, by a culture under S-limitation increases when sulfur limitation occurs; see Figure 4.

Thus, the methods of the present invention provide for advantageous effects, particularly for an increase of the yield of the desired nucleic acid, high quality of the desired nucleic acid and/or a beneficial ratio of the desired nucleic acid versus cell dry mass. Each of these advantageous effects decreases nucleic acid manufacturing costs and/or improves the effectiveness of downstream processes, such as the purification of the desired nucleic acid.

"Increasing the production of a nucleic acid" when used herein means that the production of a nucleic acid by a host cell in cell culture which is subject to a limitation of the total sulfur amount in the culture medium over the course of cell culturing as described herein results in a higher yield of said nucleic acid in comparison to a host cell, preferably an identical host cell which is, however, preferably not subject to a limitation of the total sulfur amount in the culture medium over the course of cell culturing. Put differently, a preferably identical host cell is not limited in terms of total sulfur over the course of cell culturing in comparison to a host cell as described herein which is limited in terms of total sulfur over the course of cell culturing as described herein. "A higher yield" includes the yield of a desired nucleic acid, the volumetric yield or the quality of a nucleic acid, e.g. the amount of ccc DNA in case of a plasmid. Such a yield may be, in relative terms, at least abouit 1%, 2%, 3%, 4%, 5%, 10%, 15%, 20%, 25%, 30% higher when comparing the yield of a host cell applied in a method as described herein and which is subject to sulfur limitation in comparison to a reference host cell as described herein.

Means and methods to determine the amount of dry cell matter and elemental composition of the cells, e.g. host cells as referred to herein, contained therein are well known in the art; see, e.g., Heldal etal., Applied and Environmental Microbiology (1985), 50(5): 1251-1257 or Neidhardt, F. C. (2006), Escherichia coli and Salmonella typhimurium, Cellular and Molecular Biology, Part I, Chapter 2 "Chemical Composition of Escherichia coli" (ISBN 0-914826-89-1). Alternatively, host cells may be grown with three different sulfur amounts until maximal OD600 is reached, followed by linear regression which allows calculation of sulfur amount per OD unit or dry weight of host cells. Maximum OD600 is reached when the OD600 varies over time at most within +0.5 OD600 to -0.5 OD600 units.

For doing so, the skilled person knows the amount of the C-source, e.g. glucose in the medium. In case of the condition, where sulfur is not limited, the skilled person either knows or is readily in a position to determine the yield coefficient, e.g. in a chemostat bioreactor in steady state. Accordingly, the skilled person is able to determine the (expected) biomass. He can thus also determine the maximal OD600.

In parallel, the skilled person sets up two further conditions with lower amounts of sulfur than sulfur used in the non-limiting sulfur condition, whereby the amount of C-source, e.g. glucose is identical to the amount of glucose used in the non-limiting sulfur condition. As explained above, when maximal OD600 is reached, linear regression follows which allows calculation of the sulfur amount per OD unit or dry weight of host cells.

It has been surprisingly found in the context of the present invention, that culturing host cells while limiting the available amount of sulfur in the culture medium leads to significant increase in the production of nucleic acids. Particularly, as has been found in context of the present invention, limiting the amount of sulfur for growing a desired amount of host cells comprising a nucleic acid molecule to be produced to the amount of sulfur contained in the same amount of reference host cells (preferably not comprising said nucleic acid molecule) which was grown in the same culture medium, except that there was no limitation of sulfur, leads to significant increase in the production of nucleic acids. For example, in accordance with the present invention, if an amount of X grams (dry weight) reference host cells (grown without sulfur limitation) was produced, and said X grams of reference host cells contained 10g sulfur, and it is desired to produce also X grams of host cells comprising a nucleic acid molecule to be produced in accordance with the method provided and described herein, then the amount of sulfur in the medium for culturing said host cell comprising said nucleic acid molecule is limited to about 10g or less.

The present invention thus provides an efficient and simple method for increasing the production of desired nucleic acids, thereby even reducing the amount of resources needed to produce the nucleic acids. Without being bound by theory, in accordance with the present invention, it is assumed that by limiting the sulfur amount in the culture medium the metabolism of the host cell is decreased in a manner allowing the host cell to increase production of nucleic acids molecules, thereby decoupling metabolism from nucleic acid production.

Preferably, in the method described and provided in context of the present invention, the step of limiting may comprise
(a1) predetermining the amount of dry cell weight of said host cell which is desired to be obtained in said cell culture; and
(b1) adding at the start of the cell culture, a total sulfur amount per each gram predetermined dry weight of said host cell (i.e. dry weight which is desired to be obtained) to said cell culture, which total sulfur amount is about equal to or less than the amount of sulfur contained per each gram dry weight of said reference host cell.

The above method, without being limited thereto, may be performed as follows: at the start of the cell culture a total sulfur amount is added per each gram predetermined dry weight of host cells such that sulfur becomes limiting when said host cells reached the predetermined dry cell weight. In practice - a skilled person predetermines the dry weight of host cells which he wants to have available for producing a desired nucleic acid, once metabolism is decoupled from the production of the desired nucleic acid - due to the limitation of sulfur in said host cells in line with the teaching of the present application. For this, host cells are grown without sulfur limitation until a predetermined dry cell weight is reached. Since a skilled person is able to determine dry weight of so-grown host cells and a skilled person is able to determine the amount of sulfur contained by so-grown host cells, he knows how much total sulfur he will have to add to host cells at the start of the cell culture such that sulfur becomes ideally limiting once said host cells have reached the predetermined dry cell weight. Alternatively, as described herein, host cells may be grown with three different sulfur amounts until maximal OD₆₀₀ is reached, followed by linear regression which allows calculation of sulfur amount per OD unit or dry weight of host cells. If so, metabolism will be decoupled from the production of the desired nucleic acid as is described and taught herein.

Alternatively, but also preferably, in the method described and provided in context of the present invention, the step of limiting may comprise
(a2) predetermining the amount of dry cell weight of said host cell which is desired to be obtained in said cell culture; and
(b2) reducing over the course of cell culture the amount of available total sulfur per each gram dry weight of said host cell to said cell culture, to at least or less than the amount of sulfur contained per each gram dry weight of said reference host cell.

For performing alternative (a2, b2), the skilled person, like in the above alternative (a1, b1) predetermines the dry cell weight, amount of sulfur as described above. However, rather than adding at the start of the cell culture a total sulfur amount per each gram predetermined dry weight of said host cell, sufficient sulfur, i.e., sulfur is not a limiting factor, is added such that sulfur is not limiting. If desired, the addition of sulfur is reduced over the course of cell culture. For example, if 10g total sulfur were needed for a predetermined dry cell weight of host cells, this amount is not added in total at the start of the cell culture, but added over a certain course of time, to reduce the ratio of available sulfur to dry weight of the host cells over time, thereby rendering sulfur to become a limiting factor.

Still alternatively, but preferably, in the method described and provided in context of the present invention, the step of limiting may comprise
(a3) predetermining the amount of dry cell weight of said host cell which is desired to be obtained in said cell culture; and
(b3) adding during the cell culture - from the start until the desired amount of host cells comprising said nucleic acid has been obtained-, a total sulfur amount per each gram predetermined dry weight of said host cell (i.e. dry weight which is desired to be obtained) to said cell culture, which total sulfur amount is about equal to or less than the amount of sulfur contained per each gram dry weight of said reference host cell.

For performing alternative (a3, b3), the skilled person, like in the above alternative (a1, b1) or (a2, b2) predetermines the dry cell weight, amount of sulfur as described above. However, sulfur is added from the start until the desired amount of host cells is reached. For example, if 10g sulfur were required, these 10g are added during the cell culture until the desired amount of host cells is achieved and then no more sulfur is added.

The first alternative, i.e. (a1), (b1) is more preferred; see also the Examples.

Preferably, in the method described and provided in context of the present invention, the amount of sulfur contained per each gram dry weight of said reference host cell may be about equal to or less than the amount of sulfur contained in the elemental composition per each gram dry weight of said reference host cell. In this context, the term "elemental composition" as used herein comprised the composition of elements in a host cell, including sulfur and optionally further elements such as, e.g., sodium, magnesium, phosphorus, chloride, potassium, calcium, and/or others.

Generally, in context of the present invention, sulfur may be added to a medium or a cell culture in all suitable forms which allow cultivation of cells, preferably in a form which allows uptake of the sulfur into the cells. For example, sulfur may be added in form of (hydrated) sulfate, e.g., MgSO₄, MnSO₄, FeSO₄, CuSO₄, ZnSO₄, or the respective hydrates forms, or in the form of dextran sulfate.

Preferably, in the method described and provided in context of the present invention, feeding of the cell culture with nutrients (other than sulfur) can be conducted linearly, while limiting the total sulfur amount is limited. However, feeding of the cell culture with nutrients (other than sulfur) can alternatively be conducted exponentially, while limiting the total sulfur amount is limited. As already described herein (cf., e.g., (b1), (b2) and (b3) above), limitation of the sulfur amount may be achieved by controlling the amount of sulfur added to the cell culture at the start of the cell culture, and/or during the cell culture so that the total sulfur amount is about equal to or less than the amount of sulfur contained per each gram dry weight of said reference host cell as described herein.

Sometimes, it may be required to add sulfur, e.g. in the form of a sulfur salt, e.g. MgSO4 stepwise over a certain time range into the medium due to solubility of such a sulfur salt. Accordingly, when used herein "start of the cell culture" encompasses a time range spanning 0 to 24 hours, preferably 0 to 20 hours, more preferably 0 to 16 hours at or after the start of the cell culture, respectively, during which a total sulfur amount per each gram predetermined dry weight of said host cell (i.e. dry weight which is desired to be obtained) is added to said cell culture as described herein.

A "reference host cell" as used herein, in particular in the context of the methods of the present application, is a host cell which is identical to a host as that applied in the methods described herein for increasing the production of a desired nucleic acid, The term "identical" preferably refers to another cell of the same type, species and/or strain as the host cell comprising the nucleic acid to be produced. However, said reference host cell is not subject to sulfur limitation, particularly when applied in the methods of the present application.

Preferably, said reference host cell may be grown in the same medium as the host cell applied in the methods described herein for increasing the production of a desired nucleic acid. The term "same medium" preferably relates to a cell culture medium which has the same composition of ingredients as that of the cell culture medium used to grow the host cell comprising the nucleic acid to be produced, except for the sulfur content.

Preferably, said reference host cell may be grown under the same conditions, e.g. regarding temperature, pressure, gas environment, and/or mixing rate as said host cell applied in the methods described herein for increasing the production of a desired nucleic acid.

Preferably, said reference host cell does not comprise the desired nucleic acid, particularly when applied in the methods of the present application. However, in the alternative, said reference host cell may preferably comprise the desired nucleic acid, particularly when applied in the methods of the present application. The latter alternative is preferred over the first alternative.

Hence, a preferred reference host cell is not subject to sulfur limitation and does not comprise the desired nucleic acid, particularly when applied in the methods of the present application. Alternatively, however, a preferred reference host cell is not subject to sulfur limitation and comprises the desired nucleic acid, particularly when applied in the methods of the present application.

The latter mentioned alternative of a reference host cell is preferred over the first mentioned alternative of a reference host cell.

Accordingly, a more preferred reference host cell
(i) is identical - as described herein - to a host as that applied in the methods described herein for increasing the production of a desired nucleic acid;
(ii) is not subject to sulfur limitation, particularly when applied in the methods of the present application; and
(iii) comprises the desired nucleic acid, particularly when applied in the methods of the present application.

Further, said more preferred reference host cell
(iv) may be grown in the same medium as the host cell applied in the methods described herein for increasing the production of a desired nucleic acid; and/or
(v) may be grown under the same conditions, e.g. regarding temperature, pressure, gas environment, and/or mixing rate as said host cell applied in the methods described herein for increasing the production of a desired nucleic acid.

Such a more preferred reference host cell may also be referred to herein as "control", e.g. "control host cell", "control culture", or - in the Examples - as "control bacterial culture" or the like.

Preferably, in the method described and provided in context of the present invention, predetermining the dry cell weight of said host cell may be achieved by obtaining a defined volume of a sample of said cell culture medium, harvesting said host cells, drying said host cells, and weighing said dried host cells. Means and methods to determine the amount of dry cell matter and elemental content of the cells contained therein are well known in the art; see, e.g., Heldal et al., Applied and Environmental Microbiology (1985), 50(5): 1251-1257. Preferably, in the method described and provided in context of the present invention, limiting the total sulfur amount in the culture medium of said cell culture may occur when said host cells are in log phase. Means and methods for determining whether a cell culture is in log phase are known in the art including measurement of the optical density (OD) of the cell culture; cf. e.g., https://www.implen.de/od600-diluphotometer/od600/.

Preferably in the method described and provided in context of the present invention, limiting the total sulfur amount in the culture medium of said cell culture may occur when said host cells have a dry weight of at least about 5 g per liter culture medium culture, preferably at least about 10 g, or at least about 20 g per liter culture medium.

Preferably, in the method described and provided in context of the present invention, said extrachromosomal nucleic acid may be a bacmid, cosmid, plasmid or minicircle. A plasmid may be circular or linear. If circular, a plasmid is preferably covalently, closed, circular (ccc).

Preferably, a plasmid may be a low, medium or high copy number plasmid, with a high copy number plasmid being preferred. Examples of high copy number plasmids are plasmids with a pMB1 derivative origin of replication (ori), ColE1 ori, pUC or F1 ori. High copy number plasmids with a pUC ori are preferred.

Preferably, in the method described and provided in context of the present invention, said host cell may be a eukaryotic (e.g., fungal) or prokaryotic (e.g., bacterial) host cell, with a prokaryotic cell being preferred. A preferred prokaryotic cell is an E. coli cell.

In context of the present invention, examples for eukaryotic host cells include fungal cells such as, e.g., yeast, and microscopic algae (e.g., chlorella) and protozoa.

The term "host cell", as used herein, preferably refers to a cell, into which a nucleic acid molecule which is naturally not comprised by said host cell has been (or, in context of reference host cells, can be) introduced, i.e. which has been (or, in context of reference host cells, can be) genetically-engineered. A preferred example of a prokaryotic host cell is *E. coli.* However, also *Pseudomonas species, Salmonella species, Bacillus species, Lactobacillus species, Corynebacterium species, Microbacterium species* or *Actinomycetes* species may be envisaged in context of the present invention. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell, preferably grown in culture. In a preferred embodiment or the present invention, a bacterial host cell is *E. coli.* In a more preferred embodiment, the bacterial host cell is *E. coli* K-lineage such as *E. coli* DH5a or more preferably *E. coli* JM108. The E. coli may also be from the B-lineage such as *E. coli* BL21, REL606 or the W-lineage or any other derivative of E. coli. The bacterial host cells described herein may be created by means and methods commonly known in the art.

Preferably, a prokaryotic host cell may have an inactive recA and/or endA gene. Inactivation of recA and/or endA may be due to full or partial deletion or due to a mutation. The skilled person is aware to find the respective recA and/or endA gene in a prokaryotic host by, e.g. using E. coli recA or endA, respectively, as query sequence in databases or as a probe in wet-lab experiments.

Preferably, in the method described and provided in context of the present invention, said host cell may be a recombinant host cell.

A skilled artisan is aware of genetic engineering techniques known in the art in order to generate a bacterial host cell for use in the system or process of the invention. For example, various kits are available for genetic engineering of bacterial host cell for the integration of nucleic acids comprising nucleotide sequences into a bacterial genome, either randomly or targeted; see e.g. Zhang et al. (1998), Nature Genetics 20, 123-128 or Sharan et al. (2009), Nature Protocols 4(2), 206-223. A skilled artisan is further aware of techniques for the transformation of bacterial host cell as well as with any other cloning technique which he can use for the generation of extrachromosomal elements such as plasmids, cosmids, bacmids, minicircles, etc.

In one embodiment of the present invention, the method described and provided in context of the present invention may be a method for fed-batch or continuously culturing said host cell in a fermenter.

In fed-batch culturing or continuously culturing host cells as described herein, the feed may be linear or exponential. Thus, fed-batch culturing as described herein may comprise linear feeding or exponential feeding. Likewise, continuously culturing as described herein may comprise linear feeding or exponential feeding.

The method described and provided in context of the present invention is based on the uncoupling of the growth ("growth decoupling") of the host cell from the production of a nucleic acid of interest in a two-stage process. In the inventive system and process of the invention, the growth, i.e. the propagation of the host cells, may be spatially separated from the production of the nucleic acid of interest. In one bioreactor, the seed bioreactor, the host cells may be kept under conditions that are ideal for their propagation. No or only little production of the nucleic acid of interest would be induced. Host cells from the seed reactor may be continuously transferred into a second bioreactor, the production bioreactor. The growth of the host cell can then be inhibited and the production of the nucleic acid of interest may be induced in the production bioreactor. This concept is referred to herein as "growth decoupling". This allows a continuous production. A fraction of the host cells or the supernatant/culture medium of the production reaction may be continuously removed from the production bioreactor and the nucleic acid of interest can be harvested from the host cells. The fraction that is removed from the production bioreactor may be preferably continuously replaced by fresh host cells from the seed bioreactor. This technology therefore allows the continuous production of nucleic acids of interest in rather small bioreactors with still high yield/productivity and therefore improved S-T-Y (space-time-yield) of nucleic acids of interest.

Harvesting and isolation of nucleic acids such as plasmid DNA is well-known to a person skilled in the art. In general, the production of the nucleic acid of the invention includes the growth of the microbial/bacterial culture making use of the system or process of the invention, which is optionally followed by harvesting and lysis of the microbial/bacterial host cells and optionally the purification of nucleic acid molecules, e.g., plasmid DNA. The first step of the processing usually is disintegrating the microbial/bacterial host cells by lysis, preferably alkaline lysis. The lysis reaction, in a preferred embodiment of the present invention, is performed according to methods known in the art, using an alkaline lysis solution that contains a detergent. A typical lysing solution consists of NaOH (0.2 M) and SOS (1 %), but also other alkaline solutions and other detergents can be used (see e.g. WO 97/29190). The lysing is followed by neutralization of the alkaline solution. Also this step may, in principle, be performed according to methods known per se, preferably according to methods that are gentle and can be run in a continuous and automated mode. In a preferred embodiment, in the neutralization step the lysed cell solution is mixed with the neutralizing solution. Typically, a buffered solution with acidic pH and high salt concentration is used for neutralization. Preferable this solution consists of 3 M potassium acetate (KAc) at pH 5.5. But also other neutralizing salts can be used or added. The nucleic acid molecule, e.g., plasmid DNA may then be precipitated by adding an aqueous solution comprising about 70% ethanol. By centrifugation, the nucleic acids may be pelleted and washed by aspirating the supernatant, resuspending the nucleic acid comprising pellet in the aqueous solution comprising about 70% ethanol followed by pelleting by centrifugation again. After washing, the pelleted nucleic acid may be dried.

Alternatively, or additionally to this process, also a nucleic-acid binding resin in form of a column may be used. Before capturing/purification by means of a resin, it may be necessary to adjust the parameters of the solution (like salt composition, conductivity, pH-value) to ensure binding of the desired biomolecule to the chromatographic support, usually a resin (this step is, in the meaning of the present invention, termed "conditioning step"). The simplest conditioning procedure is dilution of the cleared lysate with water or low salt buffer, especially in case the chromatographic resin in the subsequent capture step is achieved by anion exchange chromatography (WO 97/29190). Furthermore, in particular when hydrophobic interaction chromatography is used as first purification step, a high concentration salt solution may be added and the possibly resulting precipitate (which is present if a certain salt concentration in the solution is exceeded) separated by filtration or centrifugation (WO 02/04027). In case ammonium sulfate is used in high concentrations; this treatment reduces the RNA content (WO 98/11208). For capturing and purification, several steps are applied to obtain a highly purified biomolecule which meets the requirements for pharmaceuticals. As for the previous steps, enzymes, detergents and organic solvents should be avoided. Isolation and purification are performed according to methods known in the art, in particular by a combination of different chromatographic techniques (anion exchange chromatography AIEC, hydrophobic interaction chromatography HIC, size exclusion chromatography (SEC), ultra(dia)filtration, filtration or precipitation and extraction. A method that may advantageously be used, in particular for obtaining plasmid DNA (pDNA) for therapeutic applications, comprises a combination of two steps that are based on different chromatographic principles, in which either of the two steps is selected from hydrophobic interaction chromatography (HIC), polar interaction chromatography (PIC) and anion exchange chromatography (AIEC) and in which at least in one of the two steps, preferably in both steps, the chromatographic support is a porous monolithic bed, preferably a rigid methacrylate-based monolith in the form of a monolithic column. Suitable monolithic columns are commercially available under the trademark CIMmultus^{®} from BIA Separations/Sartorius). This purification process may advantageously be performed with a chromatographic support in the form of a single monolithic bed comprising a tube-in-a-tube system, the outer and inner tube carrying different functional moieties. In such a system one of the monolithic tubes represents the support for the chromatographic principle of one step and the other tube represents the support for the chromatographic principle of the other step. Preferably, the capturing/purification step can be operated in a batch wise mode or in a quasi-continuous or continuous mode, employing technologies such as annular chromatography, carousel chromatography or a simulated moving.

"Bioreactor" or "reactor", including the "seed bioreactor" or the "production bioreactor", as used herein refers to a reaction vessel for fermentation for production of cells and biosynthetic products, which may range in size from benchtop fermenters to industrial tanks. Bioreactors preferably allow automatic regulation of the flow of oxygen, culture medium and other nutrients, and maintaining the temperature and pH; they preferably minimize the potential for contamination and can produce a higher density of cells than can be produced in traditional cultures.

"Seed (bio)reactor" as used herein in the process and the system of the invention relates to the bioreactor that is used for propagation of the host cell. In the seed bioreactor, the microbial or bacterial host cell is in an uninduced state with respect to sulfur limitation which inhibits growth of said host cell. Otherwise, propagation in the seed bioreactor could not take place.

"Production (bio)reactor" as used herein in the process and the system of the invention relates to the bioreactor that is used for the production of nucleic acid molecules of interest.

Bioreactors often comprise a variety of sensors that monitor important parameters such as pH, dissolved oxygen or temperature and means for controlling these parameters. Accordingly, the seed bioreactor preferably comprises a means for regulating pH. The at least one production bioreactor preferably comprises a means for regulating pH. Means for regulating the pH may include a pH probe, a device for recording the signal of the pH probe, and/or a device for adding acid or base to the culture medium, preferably for adding base. The seed bioreactor preferably comprises means for regulating dissolved oxygen. The at least one production bioreactor preferably comprises means for regulating dissolved oxygen. Means for regulating dissolved oxygen may comprise a pO₂ probe, a control system for regulating pO₂ by pressure, ventilation rate, addition of oxygen, control of stirring and/or the like. The seed bioreactor preferably comprises means for regulating temperature. The at least one production bioreactor preferably comprises means for regulating temperature. Means for regulating the temperature may comprise a thermometer, a heating device, a cooling device, wherein the temperature control by the heating and/or the cooling device preferably is carried out by heating and/or cooling the mantle of the bioreactor. The seed bioreactor preferably comprises a gas inlet and a gas outlet and a means for regulating the gas flow. The at least one production bioreactor preferably comprises a gas inlet and a gas outlet and a means for regulating the gas flow. The at least one production bioreactor preferably comprises a biomass sensor. The biomass concentration may be regulated in the seed bioreactor by feed inflow and/or biomass outflow. The biomass concentration may be regulated in the at least one production bioreactor by feed inflow, biomass inflow, and/or biomass outflow. The gas flow may be regulated in the at least one production bioreactor. The gas flow may be regulated in the at least one seed bioreactor. The seed and the production bioreactor(s) are linked or coupled to allow the transfer of microbial or bacterial host cells from the seed bioreactor(s) to the production bioreactor(s). The flow of the culture medium comprising the host cells from the seed bioreactor to the production bioreactor preferably is controlled. This control can be done, e.g., by applying a chemostat or a turbidostat. Accordingly, the system of the present invention preferably further comprises (c) means for operating the seed and production bioreactors as linked chemostats or turbidostats. Preferably, the seed bioreactor outflow serves as inflow to the production bioreactor.

The bioreactors of the present invention, i.e. the seed and/or the production bioreactor, may be any bioreactor suitable for the purposes of the present invention. In one embodiment, the seed bioreactor is a stirred tank bioreactor. In one embodiment, the seed bioreactor is a plug flow bioreactor. In one embodiment, the production bioreactor is a stirred tank bioreactor. In one embodiment, the production bioreactor is a plug flow bioreactor.

Unless specified herein otherwise, as used herein in accordance with the present invention, "growth" of (host) cells as used herein generally means an increase of the cell number due to cell division.

As used herein "continuous fermentation" may relate to operating conditions, in which a liquid medium, such as a culture or nutrient medium, or a cells broth, is added to the fermenter, while culture fluid is released from the fermenter. The influx of the liquid medium is preferably constant at a certain rate or intermittently. The efflux of the culture fluid is preferably at the same rate so that the amount of liquid in the fermenter remains essentially constant. During continuous fermentation, biomass preferably remain essentially unchanged. In the steady state, the fermentation condition can be maintained, such as nutrient concentration, product concentration, constant pH, biomass according to the need. Bioreactors used for continuous fermentation can be stirred tank bioreactors or tubular bioreactors. A "continuous fermenter" as used herein is preferably a fermenter that is suitable for continuous fermentation.

For example, (initial) initiation of the continuous process according to the invention may be done as follows. Both, the seed and the production bioreactor, may be started as a batch culture followed by fed batch until the necessary biomass concentration is achieved. Thus, during the initiation or, in other words, starting of the process of the invention, the host cells preferably are (both in the seed bioreactor and the production bioreactor) in an uninduced state with respect to the limitation of sulfur in the cultivation medium. When the required cell density (e.g., log phase) is reached, the production in the production bioreactor can be induced by limiting the sulfur amount in the culture medium - in other words: The production bioreactor now is in an induced state with respect to the sulfur amount. Additionally, the continuous process can be started by transferring host cells from the seed bioreactor to the production bioreactor, e.g. by starting a flow of culture medium into the seed bioreactor, thereby creating a flow from the seed bioreactor outlet to the production bioreactor inlet. Thus, in the continuous process of the invention, the cell growth is preferably inhibited in the production bioreactor.

Likewise, as also contemplated in context of the present invention, a fed-batch approach may be applied. Fed-batch processes are generally known in the art and also exemplified herein, and generally comprise processes where different steps such as, e.g., feeding and cultivation take place in the same bioreactor and where the product (e.g., nucleic acid molecule) remains in the bioreactor until the end of the cultivation. Fed-batch fermentation may be preferred for cultivation processes where a particular amount or concentration of specific nutrients or culture ingredients shall be controlled. In one embodiment of the present invention, fed-batch fermentation is applied in the method described and provided herein.

Preferably, in the method described and provided in context of the present invention, said host cell may comprise a recombinant extrachromosomal nucleic acid (e.g., a plasmid, bacmid, cosmid or minicircle, preferably a plasmid).

In context of the present invention, the nucleic acid molecule to be produced may be a recombinant extrachromosomal nucleic acid (e.g., a plasmid, bacmid, cosmid or minicircle, preferably a plasmid). In one embodiment of the present invention, the nucleic acid (e.g., a recombinant extrachromosomal nucleic acid such as a plasmid, bacmid, cosmid or minicircle, preferably a plasmid) comprises a promoter sequence.

A "promoter sequence" as used herein is a non-coding expression control sequence preferably inserted nearby the start of the coding sequence of the expression cassette and regulates its expression. Put into a simplistic yet basically correct way, it is the interplay of the promoter with various specialized proteins called transcription factors that determine whether or not a given coding sequence may be transcribed and eventually translated into the actual protein encoded by the gene. It will be recognized by a person skilled in the art that any compatible promoter can be used for recombinant expression in host cells. The promoter itself may be preceded by an upstream activating sequence, an enhancer sequence or combination thereof. These sequences are known in the art as being any DNA sequence exhibiting a strong transcriptional activity in a cell and being derived from a gene encoding an extracellular or intracellular protein. It will also be recognized by a person skilled in the art that termination and polyadenylation sequences may suitably be derived from the same sources as the promoter.

The term "inducible promoter" as used herein refers to a promoter that regulates the expression of an operably linked gene or functional RNA in response to the presence or absence of an endogenous or exogenous stimulus. Such stimuli can be but are not limited to chemical compounds or environmental signals such as temperature shifts. Examples for inducible promoters include, but are not limited to, pBAD, OR2-0R1-PR, pltetO, pllacO, PesR, plac, lacUV, tac promoter, pPrpB, pTetO, FixK2, pltet0-1 or PcpcG2.

The nucleic acid molecule to be produced in accordance with the present invention as described and provided herein (e.g., a recombinant extrachromosomal nucleic acid such as a plasmid, bacmid, cosmid or minicircle, preferably a plasmid) may be capable of autonomous replication in a host cell (e. g., vectors having an origin of replication which functions in the host cell). The nucleic acid molecule may have a linear, circular, or supercoiled configuration and may be complexed with other nucleic acids or other material for certain purposes.

The nucleic acid molecule to be produced in accordance with the present invention as described and provided herein (e.g., a recombinant extrachromosomal nucleic acid such as a plasmid, bacmid, cosmid or minicircle, preferably a plasmid) may also be suitable as a vector. Vectors usually contain transcriptional control elements suitable to drive transcription such as e.g. promoters, enhancers, polyadenylation signals, transcription pausing or termination signals as elements of an expression cassette. For proper expression of the polypeptides, suitable translational control elements are preferably included in the vector, such as e.g. preferably optimized 5' untranslated regions leading to ribosome binding sites (RBS), e.g. a Kozak sequence for protein translation initiation, suitable for recruiting ribosomes and stop codons to terminate the translation process. In particular, the nucleotide sequence serving as the selectable marker genes as well as the nucleotide sequence encoding the protein of interest can be transcribed under the control of transcription elements present in appropriate promoters. The resultant transcripts of the selectable marker genes and that of the protein of interest harbor functional translation elements that facilitate substantial levels of protein expression (i.e. translation) and proper translation termination. In one embodiment of the invention, the selectable marker used is an auxotrophic marker gene used in a host strain containing an knock-out or deletion of said auxotrophic marker gene. The vector may comprise a poly-linker (multiple cloning site, MCS), i.e. a short segment of DNA that contains many restriction sites, a standard feature on many plasmids used for molecular cloning. Multiple cloning sites typically contain more than 5, 10, 15, 20, 25, or more than 25 restrictions sites. Restriction sites within an MCS are typically unique (i.e., they occur only once within that particular plasmid). MCSs are commonly used during procedures involving molecular cloning or sub cloning.

In context of the invention described and provided herein, one type of vector may be a plasmid, which refers to a circular double stranded DNA loop into which additional DNA segments may be introduced via ligation or by means of restriction-free cloning. Other nucleic acid molecule to be produced in accordance with the present invention as described and provided herein (e.g., a recombinant extrachromosomal nucleic acid such as a plasmid, bacmid, cosmid or minicircle, preferably a plasmid) which may be suitable as a vector include cosmids, bacterial artificial chromosomes (BAC), yeast artificial chromosomes (YAC) or minichromosomes. Another type of vector is a viral vector, wherein additional DNA segments may be ligated into the viral genome. The expression cassette may be inserted into the expression vector as a DNA construct. This DNA construct can be recombinantly made from a synthetic DNA molecule, a genomic DNA molecule, a cDNA molecule or a combination thereof. The DNA construct is preferably made by ligating the different fragments to one another according to standard techniques known in the art. The expression cassette may be part of the expression vector. Preferably, the expression vector is a DNA vector. The vector may conveniently comprise sequences that facilitate the proper expression of a gene of interest and an antibiotic resistance gene. These sequences typically comprise but are not limited to promoter sequences, transcription initiation sites, transcription termination sites, and polyadenylation functions as described herein. The expression cassettes may comprise an enhancer and/or an intron. Usually, introns are placed at the 5' end of the open reading frame. Accordingly, an intron may be comprised in the expression cassette for expressing the polypeptide of interest in order to increase the expression rate. Said intron may be located between the promoter and or promoter/enhancer element and the 5' end of the open reading frame of the polypeptide to be expressed. Several suitable introns are known in the state of the art that can be used in conjunction with the present invention. The expression cassette or vector according to the invention which is present in the host may either be integrated into the genome of the host or it may be maintained in some form extra chromosomally. Furthermore, the expression cassettes may comprise an appropriate transcription termination site. This, as continued transcription from an upstream promoter through a second transcription unit may inhibit the function of the downstream promoter, a phenomenon known as promoter occlusion or transcriptional interference. This event has been described in both prokaryotes and eukaryotes. The proper placement of transcriptional termination signals between two transcription units can prevent promoter occlusion. Transcription termination sites are well characterized and their incorporation in expression vectors has been shown to have multiple beneficial effects on gene expression.

The terms "5' " and " 3' " used herein refer to a convention used to describe features of a nucleotide sequence related to either the position of genetic elements and/or the direction of events (5' to 3'), such as e.g. transcription by RNA polymerase or translation by the ribosome which proceeds in 5' to 3' direction. Synonyms are upstream (5') and downstream (3'). Conventionally, nucleotide sequences, gene maps, vector cards and RNA sequences are drawn with 5' to 3' from left to right or the 5' to 3' direction is indicated with arrows, wherein the arrowhead points in the 3' direction. Accordingly, 5' (upstream) indicates genetic elements positioned towards the left hand side, and 3' (downstream) indicates genetic elements positioned towards the right-hand side, when following this convention.

The term "expression" as used herein may preferably mean the transcription of a nucleotide sequence. Said nucleotide sequence encodes preferably a protein. Accordingly, said term also includes the production of mRNA (as transcription product from a nucleotide sequence) and translation of this mRNA to produce the corresponding gene product, such as a polypeptide, or protein.

In another embodiment of the method described and provided in context of the present invention, said method may be a method for producing a recombinant extrachromosomal nucleic acid (e.g., plasmid, bacmid, cosmid or minicircle, preferably a plasmid).

The present invention further relates to the use of a culture medium comprising a total sulfur amount per each gram predetermined dry cell weight of a host cell, which is desired to be obtained in said cell culture, said total sulfur amount is about equal to or less than the amount of sulfur contained per each gram dry weight of a reference host cell, preferably when grown without limiting the total sulfur amount,
for increasing the production of a nucleic acid (preferably extrachromosomal nucleic acid) comprised by said host cell in cell culture.

As used herein, unless specifically defined otherwise, the terms "nucleic acid" or "nucleic acid molecule" are used synonymously, and may inter alia also comprise "oligonucleotide", "nucleic acid strand", or the like, and mean a polymer comprising one, two, or more nucleotides, arranged in single- or double stranded nucleotide chains.

Generally, as used in accordance with the present invention, nucleic acid molecules may comprise *inter alia* DNA molecules, cDNA molecules, RNA molecules, oligonucleotide thiophosphates, substituted ribo-oligonucleotides or PNA molecules, preferably a DNA molecule or cDNA molecule. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The polynucleotide sequence may be single- or double- stranded, linear or circular, natural or synthetic, and without any size limitation. For instance, the polynucleotide sequence may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). Said polynucleotide sequence may be in the form of a vector, plasmid or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in context of the present invention. Particularly, such a fragment is a functional fragment. Examples for such functional fragments are nucleic acid molecules which can serve as primers.

In accordance with the present invention, nucleic acid molecules may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. The nucleic acid can be in any topological conformation. For instance, the nucleic acid can be single-stranded, double-stranded, triple-stranded, quadruplexed, partially double-stranded, branched, hairpinned, circular, or in a padlocked conformation.

The embodiments which characterize the present invention are described herein, shown in the Figures, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% or 2% of a given value or range, and also comprise the respective exact numeric value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, and reagents, etc., described herein and as such can vary. The terminology used herein is for the purpose of describing particular embodiments only and is not intended to limit the scope of the present invention, which is defined solely by the claims.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well-known in the art. Generally, nomenclatures used in connection with techniques of biochemistry, enzymology, molecular and cellular biology, microbiology, genetics and protein and nucleic acid chemistry and hybridization described herein are those well-known and commonly used in the art.

The methods and techniques of the present invention are generally performed according to conventional methods well-known in the art and as described in various general and more specific references that are cited and discussed throughout the present specification unless otherwise indicated. See, e. g., Sambrook et al., Molecular Cloning: A Laboratory Manual, 3rd ed., Cold Spring Harbor Laboratory Press, Cold Spring Harbor, N. Y. (2001); Ausubel et al., Current Protocols in Molecular Biology, J, Greene Publishing Associates (1992, and Supplements to 2002); Handbook of Biochemistry: Section A Proteins, Vol 1 1976 CRC Press; Handbook of Biochemistry: Section A Proteins, Vol II 1976 CRC Press. The nomenclatures used in connection with, and the laboratory procedures and techniques of, molecular and cellular biology, protein biochemistry, enzymology and medicinal and pharmaceutical chemistry described herein are those well-known and commonly used in the art.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The present invention also relates to the following items:
1. A method for increasing the production of a nucleic acid (preferably extrachromosomal nucleic acid) comprised by a host cell in cell culture, comprising the step of limiting over the course of cell culturing the total sulfur amount in the culture medium of said cell culture such that the total sulfur amount is per each gram predetermined dry cell weight of said host cell, which is desired to be obtained in said cell culture, about equal or less than the amount of sulfur contained per each gram dry weight of a reference host cell, preferably when grown without limiting the total sulfur amount, thereby increasing the production of said nucleic acid.
2. The method of item 1, wherein the step of limiting comprises
   (a1) predetermining the amount of dry cell weight of said host cell which is desired to be obtained in said cell culture; and
   (b1) adding at the start of the cell culture a total sulfur amount per each gram predetermined dry weight of said host cell to said cell culture, which total sulfur amount is about equal to or less than the amount of sulfur contained per each gram dry weight of said reference host cell.
3. The method of item 1, wherein the step of limiting comprises
   (a2) predetermining the amount of dry cell weight of said host cell which is desired to be obtained in said cell culture; and
   (b2) reducing over the course of cell culture the amount of av available total sulfur per each gram dry weight of said host cell to said cell culture, to at least or less than the amount of sulfur contained per each gram dry weight of said reference host cell.
4. The method of claim 1, wherein the step of limiting comprises
   (a3) predetermining the amount of dry cell weight of said host cell which is desired to be obtained in said cell culture; and
   (b3) adding during the cell culture - from the start until the desired amount of host cells comprising said nucleic acid has been obtained-, a total sulfur amount per each gram predetermined dry weight of said host cell to said cell culture, which total sulfur amount is about equal to or less than the amount of sulfur contained per each gram dry weight of said reference host cell.
5. The method of any one of the preceding items, wherein the amount of sulfur contained per each gram dry weight of said reference host cell is about equal or less than the amount of sulfur contained in the elemental composition per each gram dry weight of said reference host cell.
6. The method of any one of the preceding items, wherein said reference host cell is identical to said host, except for not comprising said nucleic acid.
7. The method of any one of the preceding items, wherein said reference host cell is grown in the same medium as said host cell, except for limiting the total sulfur amount.
8. The method of any one of the preceding items, wherein predetermining the dry cell weight of said host cell is achieved by obtaining a defined volume of a sample of said cell culture medium, harvesting said host cells, and drying said host cells.
9. The method of any one of the preceding items, wherein limiting the total sulfur amount in the culture medium of said cell culture occurs when said host cells are in log phase.
10. The method of any one of the preceding items, wherein limiting the total sulfur amount in the culture medium of said cell culture occurs when said host cells have a dry weight of at least about 5 g per liter culture medium culture, preferably at least about 10 g or at least about 20 g per liter culture medium.
11. The method of any one of the preceding items, wherein said extrachromosomal nucleic acid is a bacmid, cosmid, plasmid or minicircle.
12. The method of any one of the preceding items, wherein said plasmid is a high copy number plasmid, such as a high copy number plasmid with a pMB1 derivative origin of replication (ori), ColE1 ori, pUC or F1 ori.
13. The method of any one of the preceding items, wherein said host cell is a fungal or prokaryotic host cell.
14. The method of any one of the preceding items, wherein said host cell is a recombinant host cell.
15. The method of any one of the preceding items, wherein said method is a method for continuously culturing said host cell in a fermenter.
16. The method of any one of the preceding items, wherein said host cell comprises a recombinant extrachromosomal nucleic acid (e.g., a plasmid).
17. The method of any one of the preceding items, wherein said method is a method for producing a recombinant extrachromosomal nucleic acid (e.g., plasmid).
18. Use of culture medium comprising a total sulfur amount per each gram predetermined dry cell weight of a host cell, which is desired to be obtained in said cell culture, said total sulfur amount is about equal or less than the amount of sulfur contained per each gram dry weight of a reference host cell, preferably when grown without limiting the total sulfur amount
   for increasing the production of a nucleic acid (preferably extrachromosomal nucleic acid) comprised by said host cell in cell culture.

### Figures

The Figures show:
**Figure 1** shows cell dry mass (CDM) of a bacterial culture in accordance with the methods of the present invention in direct comparison to a control bacterial culture which is under no S-limitation. It can be seen that the CDM of a control culture (under no S-limitation) increases continuously, while the CDM of a culture under S-limitation decreases at around 23 hrs after start of culture. At this stage S becomes limiting and the bacterial cells do no longer gain bio mass, but produce a desired nucleic acid, e.g. a plasmid. Thus, as soon as S becomes limiting, metabolism is, so to say, decoupled from the production of a desired nucleic acid, e.g. a plasmid.

In Figure 1, "lin/S-lim" means a process where sulfur-supply was limited and a linear feed profile was used; "lin/control" means the same linear feed profile, but no sulfur-limiting conditions; "mean" reflects that the data points refer to triplicates and that the data is reflected as mean values.

**Figure 2** shows plasmid DNA (pDNA) quality of a bacterial culture in accordance with the methods of the present invention in direct comparison to a control bacterial culture which is under no S-limitation. It can be seen that the quality of pDNA produced by a culture under S-limitation significantly increases at around 25.5 hrs, while the quality if the pDNA produced by a control culture continuously decreases.

In Figure 2, "lin/S-lim" means a process where sulfur-supply was limited and a linear feed profile was used; "lin/control" means the same linear feed profile, but no sulfur-limiting conditions; "mean" reflects that the data points refer to triplicates and that the data is reflected as mean values.

**Figure 3** shows the productivity of covalently closed circular (ccc) DNA by a bacterial culture in accordance with the methods of the present invention in direct comparison to a control bacterial culture which is under no S-limitation. It can be seen that when S becomes limiting, ccc DNA productivity is significantly increased, while it decreases in the control culture which is under no S-limitation.

In Figure 3, "lin/S-lim" means a process where sulfur-supply was limited and a linear feed profile was used; "lin/control" means the same linear feed profile, but no sulfur-limiting conditions; "mean" reflects that the data points refer to triplicates and that the data is reflected as mean values.

**Figure 4** shows the volumetric yield of ccc DNA achieved by a bacterial culture in accordance with the methods of the present invention in direct comparison to a control bacterial culture which is under no S-limitation. It can be seen that when S becomes limiting, ccc DNA yield increases, while it decreases in the control culture which is under no S-limitation.

In Figure 4, "lin/S-lim" means a process where sulfur-supply was limited and a linear feed profile was used; "lin/control" means the same linear feed profile, but no sulfur-limiting conditions; "mean" reflects that the data points refer to triplicates and that the data is reflected as mean values.

The present invention is further illustrated by the following examples. Yet, the examples and specific embodiments described therein must not be construed as limiting the invention to such specific embodiments.

### Examples

### Materials and methods

### Fed-batch fermentation

For fed-batch fermentations, E. coli cells, preferably E. coli JM108, were grown in a 2.1 L (1.0 L net volume, 0.5 L batch volume) computer-controlled bioreactor (DASGIP parallel bioreactor system, Eppendorf AG, Germany). The bioreactor was equipped with a pH probe (Hamilton Bonaduz AG, Switzerland) and an optical DO probe (Hamilton Bonaduz AG). The pH was maintained at 7.0 ± 0.1 by addition of 12.5% ammonia solution (Carl Roth, Germany); the temperature was maintained at 37 ± 0.5°C. The dissolved oxygen (O₂) level was stabilized above 30% saturation by controlling stirrer speed, aeration rate and gassing composition. Foaming was suppressed by the addition of 2 mL 1:10 diluted Struktol J673A antifoam suspension (Schill+Seilacher, Germany) to the batch medium and by the automatic addition of 1:10 diluted Struktol J673A controlled by a conductivity operated level sensor. For inoculation of bioreactor, a seed-culture was used (25 mL Batch medium inoculated with 250 µL MCB in 250 mL baffled glass flasks at 37°C with shaking at 180 rpm). Seed culture was incubated until a final OD600 of 2-4 was reached and a defined volume was transferred aseptically to the bioreactor to reach an initial ODini of 0.015.

The fermentation process was designed for a final amount of 50 g CDM of which 1.51 g were obtained in a batch volume of 500 mL and 48.5 g during feed phase via the addition of another 500 mL of feed medium. The amount of glucose for the specific medium was calculated based on a yield coefficient (Yx/s) of 0.303 g/g and added as C6H12O6· H2O. For media preparation, all chemicals were purchased from Carl Roth GmbH (Germany) if not stated otherwise.

Two different media compositions were compared regarding specific pDNA-productivity: one with a limited sulfur source (S-Limitation) and one without limited sulfur source (control).

### Media for S-Limitation

According to the grams of CDM formed during batch phase, the following components were added and afterwards sterile filtered: For Batch-Medium preparation following components were added and afterwards sterile filtered: 0.05 g yeast extract (Bacto Yeast Extract), 1.00 g NH4CI, 0.12 g MgSO4*7 H2O, 2.72 g KH2PO4, 3.69 g Na2HPO4*2 H2O, 5 g Glucose-Monohydrate, 0.1 g L-Prolin, 0.1 g L-Isoleucin, 0.05 mL of a 1% Thiamine-HCI solution, 1.0 g citric acid (water free), 8.34 mL trace element solution. The sterile filtered feed medium was composed of the following components according to the grams of CDM formed during the feed phase: 3.60 g MgSO4*7 H2O, 2.72 g KH2PO4, 3.69 g Na2HOP4*2 H2O, 169.94 g Glucose-Monohydrate, 3.0 g L-Prolin, 3.0 g L-Isoleucin, 1.0 g citric acid (water free), 1.50 mL of a 1% Thiamine-HCI stock solution, 25.0 mL trace element solution, 0.42 g MgCl2*7H2O; in total 9.6 mL of a 200 g/L MgSO4*7 H2O stock solution was pulsed into the bioreactor (3.2 mL at feed start, 3.2 mL 7h after feed start and 3.2 mL 15h after feed start).

Media for control:
According to the grams of CDM formed during batch phase, the following components were added and afterwards sterile filtered: For Batch-Medium preparation following components were added and afterwards sterile filtered: 0.05 g yeast extract (Merck), 1.00 g NH4CI, 0.12 g MgSO4*7 H2O, 2.72 g KH2PO4, 3.69 g Na2HOP4*2 H2O, 5 g Glucose-Monohydrate, 0.1 g L-Prolin, 0.1 g L-Isoleucin, 0.05 mL of a 1% Thiamine-HCI solution, 1.0 g citric acid (water free), 8.34 mL trace element solution. The sterile filtered feed medium was composed of the following components according to the grams of CDM formed during the feed phase: 3.60 g MgSO4*7 H2O, 2.72 g KH2PO4, 3.69 g Na2HOP4*2 H2O,169.94 g Glucose-Monohydrate, 3.0 g L-Prolin, 3.0 g L-Isoleucin, 1.0 g citric acid (water free), 1.50 mL of a 1% Thiamine-HCI stock solution, 25.0 mL trace element solution. The trace element solution was prepared in 5M HCI and contained (g/L): 4.41 CaCl2*2H2O, 3.34 FeSO4·*7H2O, 1.43 CoCl2*6H2O, 1.03 MnSO4*H2O, 0.15 CuSO4*5H2O, 0.17 ZnSO4*7H2O.

Feeding was initiated when the culture in batch medium entered the stationary phase. A fed-batch regime with a linear substrate feed (0.26 g/min resp. 13.91 mL/h) was used for 35 h (five generations). During S-Limitation fermentations the provided sulfur was consumed 23 hours after feed start.

All cultivations were conducted in triplicates. The mean value was used for all diagrams and tables.

### Off-line analysis

The bioreactor for off-line analysis (OD600, CDM, product) was sampled during the fed batch phase. The OD600 was measured using an Ultrospec 500 pro Spectrophotometer (Amersham Biosciences, UK), diluting the samples with phosphate-buffered saline to achieve the linear range of measurement. For the determination of CDM, 1 mL of cell suspension was transferred to pre-weighed 2.0 mL reaction tubes and centrifuged for 10 min at 16,100 rcf and 4 °C with an Eppendorf 5415 R centrifuge. The supernatant was transferred to another reaction tube and stored at -20 °C for further analysis. As a washing step, the cell pellet was resuspended in 1.8 mL RO-H2O, centrifuged, and the supernatant discarded. Afterwards, the pellet was resuspended in 1.8 mL RO-H2O and finally dried at 105 °C for 24 h. Before re-weighing the reaction tubes, they were cooled to room temperature in a desiccator.

For pDNA product analysis, the sampling volume of the cell suspension corresponding to 20 mg CDM was estimated via direct measurement of the OD600. The calculated amount was transferred to 2.0 mL reaction tubes and centrifuged at 16,100 rcf and 4 °C for 10 min. The supernatant was discarded, and the cell pellets stored at -20 C.

### pDNA analytics

The content of pDNA in supercoiled (sc)-conformation was determined using AIEX-HPLC (CIMac^{™} pDNA-0.3 Analytical Column, 1.4 µm; BIA Separations d.o.o., Slovenia). For HPLC analysis cell disintegration was performed by an alkaline lysis method (Birnboim and Doly, 1979). The obtained lysate was directly analyzed by HPLC (Agilent 1100 with quaternary pump, Diode-array detector (DAD)). Values derived from three biological replicates have a coefficient of variation lower than 10%.

### Example 1 - cell dry mass (CDM)

In Figure 1 it can be seen that the CDM of a control culture (under no S-limitation) increases continuously, while the CDM of a culture under S-limitation decreases, i.e., a culture in accordance with the methods of the present invention, at around 23 hrs after start of culture. At this stage S becomes limiting and the bacterial cells do no longer gain bio mass, but produce a desired nucleic acid, e.g. a plasmid. Thus, as soon as S becomes limiting, metabolism is, so to say, decoupled from the production of a desired nucleic acid, e.g. a plasmid.

### Example 2 - plasmid DNA (pDNA) quality

In Figure 2 it can be seen that the quality of plasmid DNA (pDNA) produced by a culture under S-limitation, i.e., a culture in accordance with the methods of the present invention, significantly increases at around 25.5 hrs, while the quality of the pDNA produced by a control culture continuously decreases.

### Example 3 - productivity of covalently closed circular (ccc) DNA

Productivity of covalently closed circular (ccc) DNA by a bacterial culture in accordance with the methods of the present invention in direct comparison to a control bacterial culture which is under no S-limitation is significantly increased when S becomes limiting. In contrast in the control culture which is under no S-limitation ccc DNA productivity decreases.

### Example 4 - volumetric yield of ccc DNA

The volumetric yield of ccc DNA achieved by a bacterial culture in accordance with the methods of the present invention in direct comparison to a control bacterial culture which is under no S-limitation, increases, while it decreases in the control culture.

## Claims

1. A method for increasing the production of a nucleic acid (preferably extrachromosomal nucleic acid) comprised by a host cell in cell culture, comprising the step of limiting over the course of cell culturing the total sulfur amount in the culture medium of said cell culture such that the total sulfur amount is per each gram predetermined dry cell weight of said host cell, which is desired to be obtained in said cell culture, about equal to or less than the amount of sulfur contained per each gram dry weight of a reference host cell, preferably when grown without limiting the total sulfur amount, thereby increasing the production of said nucleic acid.

2. The method of claim 1, wherein the step of limiting comprises
(a1) predetermining the amount of dry cell weight of said host cell which is desired to be obtained in said cell culture; and
(b1) adding at the start of the cell culture a total sulfur amount per each gram predetermined dry weight of said host cell to said cell culture, which total sulfur amount is about equal to or less than the amount of sulfur contained per each gram dry weight of said reference host cell.

3. The method of claim 1, wherein the step of limiting comprises
(a2) predetermining the amount of dry cell weight of said host cell which is desired to be obtained in said cell culture; and
(b2) reducing over the course of cell culture the amount of available total sulfur per each gram dry weight of said host cell to said cell culture, to at least or less than the amount of sulfur contained per each gram dry weight of said reference host cell.

4. The method of claim 1, wherein the step of limiting comprises
(a3) predetermining the amount of dry cell weight of said host cell which is desired to be obtained in said cell culture; and
(b3) adding during the cell culture - from the start until the desired amount of host cells comprising said nucleic acid has been obtained-, a total sulfur amount per each gram predetermined dry weight of said host cell to said cell culture, which total sulfur amount is about equal to or less than the amount of sulfur contained per each gram dry weight of said reference host cell.

5. The method of any one of the preceding claims, wherein the amount of sulfur contained per each gram dry weight of said reference host cell is about equal to or less than the amount of sulfur contained in the elemental composition per each gram dry weight of said reference host cell.

6. The method of any one of the preceding claims, wherein said reference host cell is identical to said host cell, except for not comprising said nucleic acid.

7. The method of any one of the preceding claims, wherein said reference host cell is grown in the same medium as said host cell, except for limiting the total sulfur amount.

8. The method of any one of the preceding claims, wherein predetermining the dry cell weight of said host cell is achieved by obtaining a defined volume of a sample of said cell culture medium, harvesting said host cells, and drying said host cells.

9. The method of any one of the preceding claims, wherein limiting the total sulfur amount in the culture medium of said cell culture occurs when said host cells are in log phase.

10. The method of any one of the preceding claims, wherein limiting the total sulfur amount in the culture medium of said cell culture occurs when said host cells have a dry weight of at least about 5 g per liter culture medium culture, preferably at least about 10 g or at least about 20 g per liter culture medium.

11. The method of any one of the preceding claims, wherein said extrachromosomal nucleic acid is a bacmid, cosmid, plasmid or minicircle.

12. The method of any one of the preceding claims, wherein said host cell is a fungal or prokaryotic host cell.

13. The method of any one of the preceding claims, wherein said method is a method for continuously culturing said host cell in a fermenter.

14. The method of any one of the preceding claims, wherein said host cell comprises a recombinant extrachromosomal nucleic acid (e.g., a plasmid).

15. Use of a culture medium comprising a total sulfur amount per each gram predetermined dry cell weight of a host cell, which is desired to be obtained in said cell culture, said total sulfur amount is about equal to or less than the amount of sulfur contained per each gram dry weight of a reference host cell, preferably when grown without limiting the total sulfur amount,
for increasing the production of a nucleic acid (preferably extrachromosomal nucleic acid) comprised by said host cell in cell culture.
